# EUROPEAN PATENT APPLICATION

(11) **EP 3 646 893 A1**
(43) Date of publication of application: **06.05.2020**
(21) Application number: 18203080.9
(22) Date of filing: 29.10.2018
(51) Int. Cl.: A61L 26/00, B05B 11/00, B05B 7/14

(54) **SOLID SPRAY COMPOSITION**

(71) Applicant: SOLVAY SA, 1120 Brussels (BE)
(72) Inventor: LEMAIRE, Arnaud, 5310 Noville-sur-Méhaigne (BE)

(57) **Abstract**

A solid spray composition comprising a solid peroxide, a solid acid and a metal.

## Description

The present invention relates to a solid spray composition and more particularly, to a solid spray composition for wound healing.

An increasing number of people are suffering from chronic wounds, for instance diabetic patients. The problem with this type of wounds is that the concerned tissues are generally depleted in oxygen i.e. they are hypoxic tissues (tissues in ischemia) which need to be provided with oxygen for the healing process to be completed.

Among the solutions to this problem, are worth mentioning:
- GHT (Global Hyperbaric Therapy)
- THT (Topical Hyperbaric Therapy)
- O₂ or Oxygen Breathing
- NATROX® and EPIFLOW® (Pumping system delivering O₂ over a wound)
- Granulox ® (haemoglobin spray)
- wound dressings or implants comprising oxygen generating compounds or devices.

WO 2008/124126 relates to a method of treating hypoxic tissue in vivo
comprising contacting the hypoxic tissue with a composition comprising a biodegradable polymer and an inorganic peroxide incorporated into the polymer, preferably in solid form. In some embodiments, the composition is in the form of a sheet material, and the contacting step is carried out by contacting the sheet material to the tissue. In some embodiments, the composition is in the form of injectable microparticles, and the contacting step is carried out by infecting the microparticles into the tissue. In some embodiments, the composition is in the form of a spray, and the contacting step is carried out by spraying the
composition onto the tissue. In some embodiments, the composition is in the form of a surgical or paramedical aid, and the contacting step is carried out by contacting the aid to the tissue.

As far as topical applications are concerned, the spray alternative offers the advantage of covering the wound with a uniform and thin layer of the composition, of not requiring contacting the wound; and the fact that the composition is easy to apply in outdoor or other "uncomfortable" conditions, and this even by non-skilled people.

However, the spray compositions disclosed in WO 2008/124126 suffer from the drawback that they include several non-active ingredients like a biodegradable polymer matrix and a liquid carrier. These ingredients increase the cost and weight of the spray devices through which the compositions are delivered. Besides, they tend to decrease the activity of the compositions which actually slowly releases oxygen.

The present invention aims at solving these problems by providing a spray composition for wound healing, a delivery device therefor and a wound treatment method using this composition, having increased efficiency, favourable economics and weight and size savings.

To this end, the present invention concerns a solid spray composition comprising a solid peroxide, a solid acid and a metal. It also concerns a device for delivering this solid spray composition to a wound, and a method for treating a wound by applying this solid spray composition thereto.

The idea behind the present invention is to use an acid in order to control the pH (reduce it in order to make it skin friendly) and to favour the generation of hydrogen peroxide when the composition of the invention is contacted with the moist of the wound. However, acidification tends to lower the kinetics of hydrogen peroxide decomposition (generating the oxygen feeding the hypoxic tissue) so that a metal must be added in order to catalyse said decomposition. If no acid is used, the dissolution of the solid peroxide and oxygen release are dramatically slowed. By controlling the pH, diluents like the above mentioned polymer matrix and liquid carrier can be avoided without the risk of damaging the treated tissues.

The composition according to the invention is a solid spray composition i.e. a powder comprising solid particles. Its granulometry (determined by laser diffraction spectroscopy) and other properties are adapted to be sprayed i.e. to form an aerosol. Preferably, its particles are substantially spherical (please quantify by giving ranges for the shape factor) with an average diameter between 1 to 100 µm, preferably between 2 to 50 µm and even more preferably, between 3 to 30 µm.

The solid peroxide preferably is an inorganic peroxide, more preferably an alkaline metal peroxide (like sodium, potassium peroxide or lithium peroxide) or an alkaline earth metal peroxide (like calcium peroxide, magnesium peroxide, barium peroxide, zinc peroxide or strontium peroxide), or a mixture thereof. It more preferably is calcium or magnesium peroxide, most preferably calcium peroxide which has superior oxygen release and beneficial counter ions for cicatrisation purpose. A mixture of Ca, Mg and Zn peroxide might even be better for cicatrisation purposes.

The solid acid may be chosen among organic acids like lactic acid, oxalic acid, tartaric acid, citric acid, malic acid, succinic acid, phthalic acid, ascorbic acid etc. It may also be chosen among oxides which function as Lewis acids including silico-aluminates (zeolites, alumina, silico-alumino-phosphate) and sulfated zirconia. Also many transition metal oxides are suitable solid acids, including titania, zirconia and niobia. Many solid Bronsted acids are also suitable, including sulfonated polystyrene, solid phosphoric acid, niobic acid, and heteropolyoxometallates. A mixture of solid acids may also be used. Lactic acid and malic acids givs good results in the present invention because it allows an easy control of the pH.

Suitable metals for use in the invention are those that catalyse hydrogen peroxide decomposition like Cu, Mn, Mo, Ag, Fe, Ti, Al, Zn, V, Co, Zr and Nb. Iron and Copper give good results in practice and Silver might bring an additional advantage in terms of disinfection. More than one metal may be used. In a preferred embodiment, the metal is iron, copper, titanium or silver or a mixture thereof, either in insoluble solid microparticulate form, or as soluble salts. A mixture of mineral salts of metals (oligo-elements) is preferred.

If required, the composition of the invention may comprise a pH buffer, preferably a solid pH buffer for instance chosen from Good's Buffers or Phosphated Buffer Saline, or mixtures thereof. It is namely so that the pH of the composition when contacted with water (i.e. moisture of the wound) is preferably close to the physiological pH i.e. close to 7.5. In practice, it is preferable to maintain the pH between 6 and 8. This pH range can be reached by adapting the quantity of solid acid (knowing that for instance the pH of CaO₂ in contact with water is at about 12) and is preferably maintained (buffered) by adding a solid pH buffer.

The composition of the invention may also comprise other ingredients but preferably not in a high amount; for instance, the composition may comprise anti caking agents, desiccants, inorganic coatings, surface levellers and cratering reducers, anti-electrostatic agents - which all preferably are biocompatible, and in an amount of from 0 to 25wt%, preferably 0.05 to 10wt%, more preferably 0.5 to 5wt%. In particular, the composition of the invention does not comprise a polymer matrix i.e. it contains less than 50wt% polymer, preferably less than 20wt% polymer, particularly less than 5wt% polymer or even is devoid of any polymer (biodegradable or not).

The composition of the invention can by sprayed by any device suitable to generate a solid aerosol i.e. a suspension of fine solid particles in air or another gas like nitrogen for instance. Therefore, the present invention also concerns a device comprising a storage chamber for the composition described above and means for spraying it onto a wound or any other hypoxic tissue. It may include a simple mechanistic device (like a push button with a spring) that delivers the composition in ambient air, or it may include a reservoir of compressed gas. If the composition is exposed to ambient air, the device preferably comprises means for drying said ambient air before it contacts the composition (for instance silica gel or any other drying substance or device).

Besides wound healing, other applications of the solid spray composition of the invention are the following: solid stock of oxygen for mountain climber, or people needing respiratory assistance without possibility of taking bottle, for instance in aerospace, submarines...; oxygen control in pounds to prevent cyanobacteria growth; decorative elements for aquarium releasing oxygen; spray on elements to bring oxygen and replacing pump; odour control in dustbins; in house mould & mushrooms removal (in undergrounds for instance); eradication of "Microbiologially-influenced corrosions" of underground pipeline networks (Desulfovibrio Vulgaris); oxygen release for dissolved oxygen content in irrigation water in hydroponic plant growth systems; oxygen release for dissolved oxygen content in ponds or recreational waters to reduce pond weed growth (macroalgae); shoe spray for odour control / disinfection if formulation adjusted to low pH to deliver low H2O2 concentrations; foliar fertilizer; oxygen supply to seeds; powder/spray to be applied before planting on plants roots to avoid fungus growing; stem cells therapy; disinfection and odour removal for textiles.

The present invention also concerns a method for treating a wound said method comprising spraying a layer of the composition as described above onto said wound.

Preferably, the pH is buffered during this treatment so that it remains in the range of from 6 to 8. This buffering can be obtained by using buffers as described above that are known for maintaining the pH into the natural physiological pH range.

The composition of the invention is preferably sprayed as a layer having a thickness in between 0.1 to 5 mm, more preferably between 0.5 to 3 mm, most preferably 0.75 to 2 mm.

In another embodiment of the present invention, the solid spray composition is suspended in a highly viscous hydrophilic matrix (glycerol for instance; or ethylene glycol or any other biocompatible excipient) and the suspension so obtained is wiped on a wound. This suspension preferably has a viscosity between 0,5 to 1000 Pa.s, preferably between 3 to to 500 Pa.s and more preferably between 5 to 100 Pa.s

In a preferred embodiment, the method of the invention comprises applying a wound dressing on the layer of the composition that has been sprayed or wiped onto the wound preferably a wound dressing that is able to maintain a moisture level at an optimal point in the vicinity of the wound. For instance, if the wound is dray, the wound dressing will preferably comprise a hydrogel. And is the wound is super exudative, the wound dressing will preferably be super absorbent.

## Claims

1. A solid spray composition comprising a solid peroxide, a solid acid and a metal.

2. The solid spray composition of claim 1 wherein the solid peroxide is an alkaline metal peroxide (like sodium, potassium or lithium peroxide) or an alkaline earth metal peroxide (like calcium peroxide, magnesium peroxide, barium peroxide, zinc peroxide or strontium peroxide) or a mixture thereof.

3. The solid spray composition of claim 2 wherein the solid peroxide is calcium peroxide or a mixture of Ca, Mg and Zn peroxide.

4. The solid spray composition of any of the preceding claims wherein the solid acid is chosen among organic acids (like lactic acid, oxalic acid, tartaric acid, citric acid, malic acid, succinic acid, phthalic acid, ascorbic acid etc); oxides which function as Lewis acids including silico-aluminates (zeolites, alumina, silico-alumino-phosphate) and sulfated zirconia; transition metal oxides including titania, zirconia and niobia; and solid Bronsted acids including sulfonated polystyrene, solid phosphoric acid, niobic acid, and heteropolyoxometallates.

5. The solid spray composition of claim 4 wherein the solid acid is lactic acid or malic acid.

6. The solid spray composition of any of the preceding claims wherein the metal is chosen from those that catalyse hydrogen peroxide decomposition like Cu, Mn, Mo, Ag, Fe, Ti, Al, Zn, V, Co, Zr and Nb.

7. The solid spray composition of claim 6 wherein the metal is copper, iron, titanium or silver or a mixture thereof.

8. The solid spray composition of any of the preceding claims which comprises a pH buffer, preferably a solid pH buffer.

9. The solid spray composition of claim 8 wherein the pH buffer is chosen from Good's Buffers or Phosphated Buffer Saline, or mixtures thereof.

10. A device for spraying a solid spray composition according to any of the preceding claims onto a wound, said device comprising a storage chamber for the composition and means for spraying it.

11. The device according to claim 10 wherein the composition is exposed to ambient air and wherein the device comprises means for drying the ambient air before it contacts the composition.

12. A method for treating a wound said method comprising spraying a layer of a solid spray composition as claimed in any of claims 1 to 7 onto said wound, preferably using the device according to claim 10 or 11.

13. The method according to claim 12 wherein the pH is buffered so that it remains in the range of from 6 to 8.

14. A method for treating a wound said method comprising suspending a solid spray composition as claimed in any of claims 1 to 7 in a highly viscous hydrophilic matrix and wiping the suspension so obtained on the wound.

15. The method according to any of claims 12 to 14 comprising applying a wound dressing on the layer of the solid spray composition.
